Europäisches Patentamt

European Patent Office

Office européen des brevets

(11) Publication number: **0 374 913**
**A1**

## (12) EUROPEAN PATENT APPLICATION

(21) Application number: 89123652.3

(22) Date of filing: 21.12.89

(51) Int. Cl.5: **C12N 15/81, C12N 15/53, C12P 21/02, //(C12N15/81, C12R1:84)**

The applicant has filed a statement in accordance with Rule 28 (4) EPC (issue of a sample only to an expert). Accession number(s) of the deposit(s): NRRL Y - 11430 , NRRL Y - 15851.

(30) Priority: 22.12.88 US 289357

(43) Date of publication of application:
**27.06.90 Bulletin 90/26**

(84) Designated Contracting States:
**AT BE CH DE ES FR GB GR IT LI LU NL SE**

(71) Applicant: **PHILLIPS PETROLEUM COMPANY**
**5th and Keeler**
**Bartlesville Oklahoma 74004(US)**

(72) Inventor: **Digan, Mary Ellen**
**178 Centre Street No. 16**
**Mountain View, CA 94041(US)**

(74) Representative: **Dost, Wolfgang,**
**Dr.rer.nat.,Dipl.-Chem. et al**
**Patent- & Rechtsanwälte Bardehle .**
**Pagenberg . Dost . Altenburg . Frohwitter .**
**Geissler & Partner Galileiplatz 1 Postfach 86**
**06 20**
**D-8000 München 86(DE)**

(54) Pichia pastoris glyceraldehyde-3-phosphate dehydrogenase gene.

(57) The present invention discloses the Pichia pastoris glyceraldehyde-3-phosphate dehydrogenase gene, and fragments thereof.

FIG. 1

## PICHIA PASTORIS GLYCERALDEHYDE-3-PHOSPHATE DEHYDROGENASE GENE

This invention relates to the field of recombinant DNA biotechnology. In one of its aspects the invention relates to novel DNA fragments containing part or all of the Pichia pastoris glyceraldehyde-3-phosphate dehydrogenase gene. In another aspect the invention relates to use of the above-described DNA fragments. In yet another aspect, the invention relates to novel vectors and cells transformed with these above-described DNA fragments.

## Background of the Invention

As recombinant DNA biotechnology has developed in recent years, the controlled production by cells of an enormous variety of useful polypeptides has become possible. Many eukaryotic polypeptides, for example human growth hormone, leukocyte interferons, human insulin and human proinsulin have been produced by various recombinant cells. The continued application of techniques already in hand is expected in the future to permit recombinant production of a variety of other useful polypeptide products.

The basic techniques employed in the field of recombinant technology are known by those of skill in the art. The elements desirably present for the practice of recombinant DNA biotechnology include, but are not limited to:

(1) a gene encoding one or more desired polypeptide(s), operably linked to a regulatory region which controls expression in the host cell, wherein operably linked refers to a juxtaposition wherein the components are configured so as to perform their usual function.

(2) a vector, usually a plasmid into which a nucleotide sequence can be inserted; a vector is any nucleotide sequence-containing construct capable of transforming a host;

(3) a suitable host into which the desired nucleotide sequence can be transferred, where the host also has the cellular apparatus to express the information encoded in the transferred nucleotide sequence.

It is particularly desirable to have 5′ regulatory regions which provide for high levels of DNA transcription to RNA and are responsive to exogenous environmental stimulus. Currently no 5′ regulatory region is known in the art which can be used with the highly productive fermentation yeast Pichia pastoris which is transcribed at a high level in response to the presence of a variety of carbon sources such as glucose, glycerol and methanol. Since glucose, glycerol and methanol are relatively inexpensive, readily available carbon sources, it would be a significant contribution to the art to isolate a regulatory region which is transcribed at a high rate in response to the presence of these carbon sources.

Thus, it is an object of this invention to provide a 5′ regulatory region transcribed at a high rate in response to a variety of carbon sources such as glucose, glycerol and methanol.

It is a further object of this invention to provide vectors containing the aforesaid 5′ regulatory region operably linked to heterologous DNA sequences which encode at least one polypeptide and methods of inducing said regulatory region to facilitate expression of heterologous DNA sequences which encode at least one polypeptide. Another object of this invention is to provide a 3′ transcription termination sequence. Still another object of the present invention is to provide the DNA sequence of the Pichia pastoris glyceraldehyde-3-phosphate dehydrogenase (GAPDH) structural gene.

Other aspects, objects and several advantages of this invention will be apparent from the foregoing specifications, examples and claims.

## Summary of the Invention

In accordance with the present invention, there is provided a novel DNA fragment consisting essentially of the Pichia pastoris GAPDH 5′ regulatory region.

A further aspect of this invention provides novel vectors comprising the GAPDH 5′ regulatory region capable of replicating in cells selected from the group consisting of prokaryotic and eukaryotic cells.

Yet another aspect of this invention provides a method for activating the GAPDH 5′ regulatory region to facilitate high level expression of heterologous DNA sequences which encode at least one polypeptide comprising contacting a suitable host cell which has been transformed with the GAPDH 5′ regulatory region operably linked to a heterologous DNA sequence with a compound selected from the group consisting of glucose, glycerol and/or methanol or combinations thereof in a manner to facilitate host cell ingestion of said compound under conditions suitable for growth of the host cell.

In still another aspect of this invention, I have also isolated a novel DNA fragment consisting essentially of the Pichia pastoris GAPDH 3′ transcription termination sequence.

In another aspect of this invention, there is provided a novel, isolated DNA fragment consisting essentially of the Pichia pastoris GAPDH structural gene.


## Brief Description of the Figures

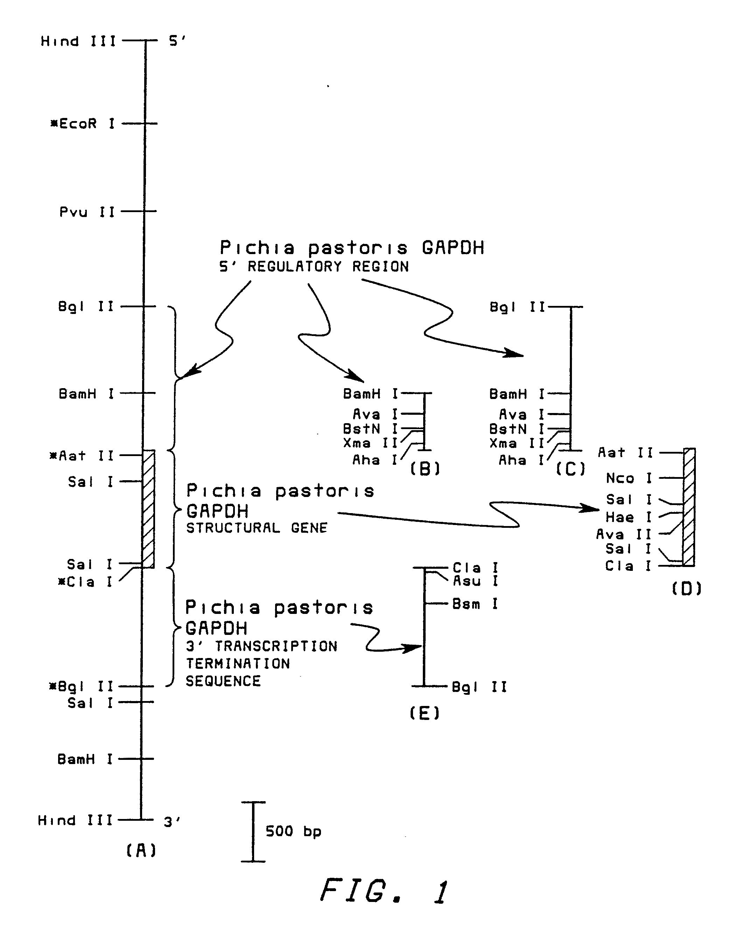

Figure 1 provides an approximate restriction map of the Pichia pastoris GAPDH gene. Restriction map A shows the location of the GAPDH 5′ regulatory regions, the GAPDH structural gene, (which encodes the GAPDH polypeptide), and the 3′ GAPDH transcription termination sequence. The asteriks in this drawing indicate restriction sites for which all the sites may not be shown within the fragment. Restriction maps B and C show two DNA fragments of the GAPDH 5′ regulatory region which are capable of effecting the transcription of DNA into RNA. Restriction fragment B has also been sequenced, it begins at about nucleotide-490 and extends to about nucleotide-1 as shown in Table 1. Restriction map D provides a map of the restriction sites present in the GAPDH structural gene. Restriction map E provides a restriction map of the GAPDH 3′ transcription termination sequence.

Figure 2 provides an outline of the construction of plasmid pRA110.

Figure 3 provides an outline of the construction of plasmid pSL19. The abbreviation, GAPDHp, designates a GAPDH 5′ regulatory region fragment.

Figure 4 provides an approximate restriction map of plasmid pSL20A. The abbreviation, GAPDHp, designates a GAPDH 5′ regulatory region fragment.

In the attached figures, restriction sites employed for manipulation of nucleotide sequences, but which are destroyed upon ligation, are indicated by enclosing the abbreviation for the destroyed site in parentheses.


## Description of the Invention

In accordance with the present invention, there is provided a novel isolated DNA fragment comprising a GAPDH gene derived from Pichia pastoris. A partial restriction map of the Pichia pastoris GAPDH gene and flanking DNA is provided in Figure 1. This gene has been further characterized by the partial nucleotide sequence which is provided in Table I.

Also provided by the present invention are novel DNA fragments comprising the GAPDH 5′ regulatory region derived from Pichia pastoris.

## Table 1

### Partial Nucleotide Sequence of the
### Pichia pastoris GAPDH Gene

```
                                                      -490
                                                      BamHI
                                                      GGATCCTTTT

-480      -470      -460      -450      -440      -430      -420
TTGTAGAAATGTCTTGGTGTCCTCGTCCAATCAGGTAGCCATCTCTGAAATATCTGGCTCCGTTGCAACT

-410      -400      -390      -380      -370      -360      -350
CCGAACGACCTGCTGGCAACGTAAAATTCTCCGGGGTAAAACTTAAATGTGGAGTAATGGAACCAGAAAC

-340      -330      -320      -310      -300      -290      -280
GTCTCTTCCCTTCTCTCTCCTTCCACCGCCCGTTACCGTCCCTAGGAAATTTTACTCTGCTGGAGAGCTT

-270      -260      -250      -240      -230      -220      -210
CTTCTACGGCCCCCTTGCAGCAATGCTCTTCCCAGCATTACGTTGCGGGTAAAACGGAAGTCGTGTACCC

-200      -190      -180      -170      -160      -150      -140
GACCTAGCAGCCCAGGGATGGAAAAGTCCCGGCCGTCGCTGGCAATAATAGCGGGCGGACGCATGTCATG

-130      -120      -110      -100      -90       -80       -70
AGATTATTGGAAACCACCAGAATCGAATATAAAAGGCGAACACCTTTCCCAATTTTGGTTTCTCCTGACC

-60       -50       -40       -30       -20       -10
                                                            ga
CAAAGACTTTAAATTTAATTTATTTGTCCCTATTTCAATCAATTGAACAACTATCAAAACACA

          10        20        30        40              50
att c                                          AatII
ATG GCT ATC ACT GTC GGT ATT AAC GGT TTC GGA CGT ATT GGA CGT CTC GTT

          60        70        80        90              100
CTG AGA GTC GCT CTT TCC AGA GCT GAC ATC AAG GTT GTT GCT ATC AAC GAC

          110       120       130       140             150
CCA TTC ATT GCT CCA GAA TAC GCT GCT TAC ATG TTC AAG TAC GAC TCT ACC

          160       170       180       190             200
CAC AAG GCT TAC AAG GGT GAG GTT TCT GCC AGC GGC AAC AAG ATC AAC ATT

          210       220       230       240             250
GAC GGT AAA GAA ATC ACC GTT TTC CAA GAG AGA GAC CCT GTC AAC ATC CCA

          260       270       280       290             300
                    SalI
TGG GGT AAG GCT GGT GTC GAC TAC GTC ATT GAG TCC ACC GGT GTT TTC ACC

          310       320       330       340             350
```

4

ACT TTG GAG GGT GCC CAA AAG CAC ATC GAC GCC GGT GCC AAG AAG GTG GTC

```
    360         370         380         390         400
ATC ACT GCT CCA TCC AAG GAT GCT CCA ATG TTC GTT GTC GGT GTC AAC

    410         420         430         440         450
GAG GAG AAA TAC ACT TCT GAC TTG AAC ATT GTC TCC AAT GCT TCT TGT ACT

    460         470         480         490         500
ACC AAC TGT TTG GCT CCA TTG GCC AAG GTT GTC AAC GAC ACT TTC GGA ATT

    510         520         530         540         550
GAG TCC GGT TTG ATG ACC ACC GTC CAC TCC ATG ACC GCC ACT CAA AAG

    560         570         580         590         600
ACC GTT GAC GGT CCA TCC CAC AAG GAC TGG AGA GGT GGT AGA ACG GCT TCT

    610         620         630         640         650
GGT AAC ATC ATT CCA TCT TCC ACT GGT GCT GCT AAG GCC GTC GGT AAG GTT

660         670         680         690         700
ATT CCA GAA TTG AAC GGT AAG CTG ACC GGT TTG GCT TTC CGT GTC CCA

    710         720         730         740         750
ACC GTC GAT GTC TCC GTT GTT GAC TTG ACC GTC AAC TTG AAG AAG GAG ACT

760         770         780         790         800
ACC TAC GAG GAG ATC AAG TCT GTT ATC AAG GCT GCT TCC GAG GGT AAG CTC

810         820         830         840         850
AAG GGT GTT TTG GGT TAC ACT GAA GAT GCC GTT GTC TCT TCT GAC TTC

    860         870         880         890         900
TTG GGT GAC GAG AGA TCC TCC ATC TTC GAC GCT TCT GCC GGT ATT CAA TTG

    910         920         930         940         950
ACT CCA TCT TTC GTC AAG CTG ATC TCT TGG TAC GAC AAC GAG TAC GGT

    960            970         980         990            1000
                         SalI
TAC TCC ACC AGA GTC GTC GAC TTG TTG CAA CAC GTT GCT AAG GCT TAA

    1010       1020       1030       1040       1050       1060       1070
ClaI
ATCGATTTGTATGTGAAATAGCTGAAATTCGAAAATTTCATTATGGCTGTATCTACTTTAGCGTATTA

    1080       1090       1100       1110       1120       1130       1140
GGCATTTGAGCATTGGCTTGAACAATGCGGGCTGTAGTGTGTCACCAAAGAAACCATTCGGGTTCGGATC

    1150       1160       1170       1180       1190       1200       1210
TGGAAGTCCTCATCACGTGATGCCGATCTCGTGTATTTTATTTTCAGATAACACCTGAAGACTTTTGGGT

    1220       1230       1240       1250       1260       1270       1280
```

CGGAGGACTGGCTCTTTCCGATCAAATTGGAATGGAAAATTGCTCCTCTAATAAAGGGGTGCCCAACACT

```
      1290      1300      1310      1320      1330      1340      1350
CTTTGTAACACAGGACACGTTTATTGCTAACTCGATTGCATTCTTTCCTTTCCCACAACCGGATCTGGTC

      1360      1370      1379
TGGTGACATCTCTCCTGTCCTTATCTAAA
```

Further in accordance with the present invention, there is provided a novel DNA fragment comprising the GAPDH 3' transcription termination sequence of the GAPDH gene derived from Pichia pastoris.

In yet another aspect of the present invention, there is provided the GAPDH structural gene which codes for the Pichia pastoris GAPDH polypeptide.

The GAPDH structural gene, GAPDH 5' regulatory region and GAPDH 3' transcription termination sequence of Pichia pastoris can be recovered from Pichia pastoris cultures such as NRRL #11430 by methods analogous to those set forth in the following examples. A general method for recovering the Pichia pastoris GAPDH gene consists of using a GAPDH probe, such as a GAPDH gene fragment from a related yeast, to screen a library of Pichia pastoris DNA in Southern blots. The 2.2kb HindIII fragment from plasmid pp6y, described by Musti et al.(1983) Gene 25, 133-143, would be a suitable probe which could be used. Other probes could be selected or synthesized based on the sequence disclosed in Table I. The Southern blots can be performed by any suitable protocol known to those skilled in the art. Pichia pastoris libraries can be prepared by techniques known in the art including but not limited to the method described by Cregg et al. (1985), Mol. Cell Bio. 5, 3376-3385. Other methods for isolating this gene from Pichia pastoris may also be utilized.

The GAPDH structural gene, GAPDH 5' regulatory region fragment (Figure 1(B)) and GAPDH 3' transcription termination sequence can also be obtained by using the nucleotide sequence provided in Table 1 and synthesizing the sequence using known enzymatic or chemical means. Suitable means include but are not limited to chemical procedures based on phosphotriester, phosphite, or cyanoethylphosphoramidite chemistry.

Those skilled in the art will also recognize that the isolated Pichia pastoris GAPDH structural gene, GAPDH 5' regulatory region or GAPDH 3' transcription termination sequence of the present invention as compared to subsequently isolated Pichia pastoris GAPDH structural gene, GAPDH 5' regulatory regions or GAPDH 3' transcription termination sequences may contain a de minimis number of nucleotide differences because of genetic drift or sequencing error which may occur.

Modification to the GAPDH structural gene, GAPDH 5' regulatory region and GAPDH 3' transcription termination sequence can also be performed such as adding linker DNA or performing M13 mutagenesis to provide new restriction sites, remove existing 5' or 3' restriction sites or blunt ending the fragments.

Once the GAPDH structural gene, GAPDH 5' regulatory region or GAPDH 3' transcription termination sequence is recovered it may be maintained or replicated in eukaryotic or prokaryotic plasmid-host systems such as pBR322 maintained in E. coli or any other suitable system known in the art.

Those skilled in the art will recognize that numerous additional DNA sequences can also be incorporated into the vector employed such as bacterial plasmid DNA, marker genes, bacteriophage DNA, autonomous replicating sequences to name only a few representative examples.

The GAPDH 5' regulatory region is contained within the DNA fragment extending from the BglII site at approximately nucleotide -1200 to about the AatII site near the beginning of the structural gene at approximately nucleotide 16 (as shown in Figure 1(A)). The 3' end of this fragment, however, can end at nucleotide-1 with no loss of effect. The GAPDH 5' regulatory region can also be recovered in approximately a 490 base pair DNA fragment beginning at the BamHI site at approximately nucleotide -490 extending to approximately nucleotide-1. For convenience a restriction site may be inserted at approximately nucleotide 1, such as the EcoRI site indicated in lower case letters in Table 1. Example VII demonstrates a method whereby an EcoRI site is inserted at approximately nucleotide 1 (indicated in lower case lettering in Table 1) utilizing m13 mutagenesis, thus providing a BamHI - EcoRI fragment which functions as a GAPDH 5' regulatory region (see Figure 1(B)). Both the BglII - AatII fragment and the BamHI - EcoRI fragment are capable of effecting the transcription of DNA to RNA when operably linked to and positioned at the 5' end of a heterologous DNA sequence coding for at least one polypeptide.

To utilize the GAPDH 5' regulatory region disclosed herein, the fragments described in Figure 1 (B or C) can each be operably linked to heterologous DNA sequences encoding at least one polypeptide. For the purpose of this specification heterologous DNA sequences are combinations of DNA sequences which do not naturally occur in association with said regulatory region. Suitable heterologous DNA sequences encoding at least one polypeptide which could be operably linked with the GAPDH 5′ regulatory region include but are not limited to streptokinase, tissue plasminogen activator, hepatitis surface antigens and bovine lysozyme. Heterologous DNA sequences used with the present invention should contain a 5′ ATG start codon, a 3′ stop codon and may additionally include nucleotide sequences which function to stabilize the mRNA, or direct polyadenylation.

The combination of the GAPDH 5′ regulatory region operably linked to a heterologous DNA sequence may be inserted in a suitable vector. Numerous yeast vector-host cell combinations are possible and are known to those skilled in the art. Additional sequences such as marker genes or other sequences which render the vector capable of growth amplification and rapid propagation in bacteria or yeast may also be present.

Suitable host cells for the use of the GAPDH 5′ regulatory region include yeast such as Saccharomyces, Pichia and Hansenula, and particularly Pichia pastoris.

Transformation of the GAPDH structural gene, GAPDH 5′ regulatory region or GAPDH 3′ transcription termination sequence into a host cell line by a compatible vector can be accomplished by suitable transformation techniques known to those skilled in the art.

The GAPDH 5′ regulatory region when operably linked to a heterologous DNA sequence which has been transformed into a suitable host will allow the transcription of DNA into RNA. Expression of heterologous DNA sequences operably linked to a regulatory region transformed into a suitable host may be induced to higher levels of expression using a method comprising contacting the transformed host with various carbon sources such as those selected from the group consisting of glucose, glycerol and/or methanol in a manner to facilitate host cell ingestion of one of the compounds listed above under conditions suitable for the growth of the host cell. Glucose and glycerol are currently preferred to induce higher levels of transcription of the heterologous DNA sequence which has been transformed into the host cell operably linked to the GAPDH 5′ regulatory region.

It is also recognized that not all yeast hosts will be capable of utilizing all carbon sources. Therefore, it will be advantageous to select yeast hosts which are capable of utilizing glucose, glycerol or methanol to obtain higher levels of transcription. Even in yeast incapable of utilizing these carbon sources, low level transcription may be observed.

Those of skill in the art will also recognize that the optimum amount of glucose, glycerol and/or methanol utilized to obtain high rates of transcription will vary from yeast to yeast depending on a variety of factors. For Pichia pastoris, it is currently believed that higher levels of transcripton may be obtained from cells transformed with the GAPDH 5′ regulatory region operably linked to a heterologous DNA sequence encoding at least one polypeptide with (a) methanol concentrations between about 0.2% to about 1.5% by weight per volume of feed, preferably about 0.5% by weight per volume of feed; (b) glucose concentrations between about 1% to about 5% by weight per volume of feed, preferably about 2% by weight per volume of feed; and (c) glycerol concentrations between about 1% to about 5% by weight per volume of feed, preferably about 2% by weight per volume of feed, wherein all other nutrient and growth requirements are adequately provided. Other yeast capable of utilizing glucose, glycerol or methanol will be expected to have different ranges over which higher levels of transcription may be obtained which may be determined by those skilled in the art.

The GAPDH 3′ transcription termination sequence of the GAPDH gene is believed to terminate mRNA transcription or stabilize mRNA when linked 3′ to a DNA sequence which encodes a polypeptide. This 3′ termination sequence has been further characterized by the partial nucleotide sequence which is provided in Table 1 from about nucleotide 1005 to about nucleotide 1380. The GAPDH 3′ transcription termination sequence is contained within the DNA fragment extending from about the ClaI site at approximately nucleotide 970 to about the BglII site at approximately nucleotide 2000 (as shown in Figure 1(E)). However, the GAPDH 3′ termination sequence need only extend about 300 base pair 3′ of the GAPDH stop codon, which is located at approximately nucleotide 1000 (as shown in Table 1) to terminate mRNA transcription. Thus the GAPDH 3′ transcription termination sequence can consist of approximately 300 to approximately 1000 nucleotides of DNA 3′ to the GAPDH structural gene. However, it has been found useful in Pichia pastoris to also include from 10-30 nucleotides 5′ to the stop codon for enhanced messenger RNA transcription termination. It is currently preferred that the GAPDH 3′ transcripton termination sequence be utilized as a ClaI - BglII fragment consisting of approximately 1000 nucleotides. The GAPDH 3′ transcription termination sequence may be operably linked to a heterologous DNA sequence which codes for a polypeptide and utilized to terminate transcription of or stabilize mRNA in yeast such as Saccharomyces,

7

Pichia and Hansenula but it is particularly well suited for use in Pichia pastoris.

The GAPDH structural gene extends from about nucleotide 1 to about nucleotide 999 (as shown in Table 1 or Figure 1(D)). The GAPDH structural gene may be utilized in recombinant biotechnology for a variety of purposes including but not limited to (a) DNA constructs for performing additive homologous recombination (a process for inserting heterologous DNA sequences into the Pichia pastoris genome at the GAPDH locus without disrupting GAPDH gene activity) and (b) the production of Pichia pastoris glyceraldehyde-3-phosphate dehydrogenase protein for assays.

The following non-limiting examples are provided to illustrate the practice of the present invention.

## Examples

## General Information

### Strains

Pichia pastoris NRRL Y-11430
Pichia pastoris GS115 (his4) NRRL Y-15851
E. coli MC1061 [F⁻araD139 Δ(araABD1C-leu) 7679ΔlacX74 galU gal K rpsL hsdR]
E. coli JM103 Δ(lacpro) thi rps1 (strA) SupE endA sbcB hsdR

### Media, Buffers, and Solutions

#### 1 M Tris buffer

121.1 g Tris base in 800 mL of $H_2O$;
adjust pH to the desired value by adding concentrated (35%) aqueous HCl; allow solution to cool to room temperature before final pH adjustment, dilute to a final volume of 1 L.

#### TE buffer

0.1 mM EDTA
in 0.01 M (pH 8.0) Tris buffer

#### SSC

0.15 M NaCl
0.15 M sodium citrate
adjusted to pH 7.0 with NaOH

#### Denhardt's solution

5 g Ficoll
5 g polyvinylpyrrolidone
5 g bovine serum albumin (BSA; Pentax Fraction V)
brought to a total volume of 500 mL with water

#### 20X SSPE

20 mM EDTA
0.2 M $NaH_2PO_4 \cdot H_2O$
3.6 M NaCl
adjusted to pH 7.4 with NaOH

LB (Luria-Bertani)medium

10 g Bacto-tryptone
5 g Bacto-yeast extract
10 g NaCl
in 1 L of water, adjusted to pH 7.5 with NaOH

YPD medium

1% Bacto-yeast extract
2% Bacto-peptone
2% Dextrose

YPD agar

YPD medium + 2% agar

YNB medium

6.75 g yeast nitrogen base without amino acids (DIFCO) in 1 L of water

SED

1 M sorbitol
25 mM EDTA
50 mM DTT

SCE buffer

9.1 g sorbitol
1.47 g sodium citrate
0.168 g EDTA
50 mL H$_2$0
--pH to 5.8 with HCl

CaS

1 M sorbitol
10 mM CaCl$_2$
--filter sterilize

PEG solution

20% polyethylene glycol-3350
10 mM CaCl$_2$
10 mM Tris•HCl (pH 7.4)
--filter sterilize

Formamide dye mix

0.1% xylene cylenol FF
0.2% bromophenol blue
10 mM EDTA
95% deionized formamide

Acrylamide stock

38 g acrylamide

9

2 g bis(N,N-methylenebisacrylamide)
add water to total volume of 100 mL

Bottom gel

14.4 g urea
3.0 g sucrose
7.5 mL 10x TBE
4.5 mL acrylamide stock
0.3 mL bromophenol blue solution (0.01 g/mL)
add water to give total volume of 30 mL

dideoxy:

dd ATP 0.125 mM
dd CTP 0.10 mM
dd GTP 0.10 mM
dd TTP 0.80 mM

DNTP stocks

0.5 mM dGTP
0.5 mM dCTP
0.5 mM TTP
0.5 mM dATP

10X Klenow Dilution Buffer

70mM Tris•HCl, pH 7.5
200mM NaCl
70mM MgCl$_2$
1mM EDTA

TBE buffer

0.089 M Tris-borate
0.089 M boric acid

SET (20X)

3 M NaCl
0.4 M Tris•Cl, pH 7.8
20mM EDTA

10X TMD pH 7.5

0.1 M Tris•HCl, pH 7.5
0.05 M MgCl$_2$
0.075 M DTT
0.01 M ATP

Unless otherwise specified, the above solutions represent the basic (1x) concentration employed. Throughout the examples, where different concentration levels are employed, that fact is indicated by referring to the solution as a multiple of the basic (1x) concentration.

Regeneration Agar

1) Agar-KCl : 9 g Bacto-agar, 13.4 g potassium chloride, 240 mL H$_2$0, autoclave.
2) 10x glucose: 20 g dextrose, 100 mL H$_2$0, autoclave.

10

3) 10x YNB: 6.75 g Yeast Nitrogen Base without amino acids, 100 mL H$_2$0, autoclave.

4) Add 30 mL of 10x glucose and 30 mL of 10x YNB to 240 mL of the melted Agar-KCI solution. Add 0.6 mL of 0.2 mg/mL biotin and any other desired amino acid or nucleic acid to a concentration of 20 μg.mL. Hold melted Regeneration Agar at 55-60° C.

Zymolyase 60,000

Source: Miles Laboratories

Sequencing

DNA sequencing was by the dideoxynucleotide chain - termination method of Sanger et al., PNAS 74, 5463 (1977).

The following abbreviations are used:

EDTA ethylenediamine tetracetic acid
TEMED N,N,N',N'-tetramethylenediamine
DTT dithiothreitol
BSA bovine serum albumin
EtBr ethidium bromide
Ci Curie
dATP deoxyadenosine triphosphate
dGTP deoxyguanosine triphosphate
dTTP deoxythymidine triphosphate
dCTP deoxycytidine triphosphate
dXTP "generic" deoxy triphosphate nucleotide

Restriction and DNA-modifying enzymes were purchased from Bethesda Research Laboratories, New England Biolabs, or Boehringer Mannheim.

## Example I

## Isolation of GAPDH Gene From Pichia pastoris

20μg of plasmid pp6y [Musti, et al., Gene 25, 133 (1983)] were digested with HindIII. A 2.2 Kb HindIII fragment was isolated from a 0.8% agarose gel and labeled by nick translation (Maniatis, et al. 1982). This fragment contained most of the coding sequence and the 5' sequence of one of the three S. cerevisiae glyceraldehyde-3-phosphate dehydrogenase (GAPDH) genes.

6 μg of genomic DNA from Pichia pastoris NRRL Y-11430 (prepared according to Example III) were digested with EcoRI or HindIII. This genomic DNA was screened by Southern filter hybridization using 1 μg of cut genomic DNA/lane and the labeled 2.2 kb HindIII fragment from above. A single band of hybridization was seen with both digests.

This probe was also used to screen a library of Pichia pastoris genomic DNA. The library has been described previously [Mol. and Cell Bio. 5, 3376 (1985)] and is a partial Sau3A digest of Pichia genomic DNA inserted into the BamHI site of YEp13 (ATCC 37115). The library was screened by transforming competent MC1061 bacterial cells (Maniatis et al., 1982), plating these cells on LB and 50 μg/ml ampicillin plates, and performing filter lifts as described in Grunstein and Hogness, PNAS 72:3961 (1975). Individual positive colonies were examined using BamHI, SalI, and PvuII digests of DNA prepared by a standard miniprep procedure (Maniatis et al., 1982). From these miniprep digests, three distinct, but overlapping, clones were identified and were designated pPGAP-6, pPGAP-7, and pPGAP-9. The partial restriction map of a composite of these is provided in Figure 1.

Restriction fragments of the genomic clones containing the GAPDH structural gene were identified by hybridization of the Saccharomyces probe to Southern blots of the clones. On these blots, an approximately 700 bp SalI fragment hybridized strongly, and the adjacent, approximately 760 bp BamHI-SalI fragment also hybridized. These fragments were isolated from a 0.8% agarose gel after digesting 40 μg of pPGAP-6 with SalI and BamHI. The 700 bp SalI fragment was subcloned into SalI-digested and alkaline phosphatase-treated M13 mp18 DNA, resulting in the M13 subclone designated G1. Similarly, the 760 bp SalI-BamHI fragment was subcloned into SalI- and BamHI-cut and alkaline phosphatase-treated M13mp18 DNA, resulting in the M13 subclone designated G15. Other DNA pieces were used for DNA sequencing. The carboxyl terminal coding and 3′ non-coding sequences were determined by sequencing template DNA from other M13 subclones. The sequences of the Pichia pastoris GAPDH structural gene and surrounding 5′ and 3′ genomic DNA is presented in Table 1. For hybridization to filter bound DNAs, purified DNAs were subjected to electrophoresis through 0.7% agarose gels buffered in 1X TBE buffer (Maniatis et al., 1982) and transferred to nitrocellulose filters using standard protocols [Southern, J. Mol. Bio. 98, 503 (1975)]. DNAs from bacterial colonies were transferred to nitrocellulose after chlormphenicol amplification using standard methods [Grunstein and Hogness, PNAS 72, 3961 (1975)]. Labeling of DNA by nick-translation was performed using standard methods (Maniatis et al., 1982). The cross-species hybridizations to genomic Southern blots or to bacterial colony transfers were in 40% formamide, 5X SSPE, 5X Denhardt's, 0.1% SDS, and 0.1% sodium pyrophosphate, and washed at 50°C in 3X SSC, 1mM EDTA, 0.1% SDS, and 0.1% sodium pyrophosphate.

## Example II

## Transformation of Pichia pastoris

Yeast cells were inoculated into about 10 ml of YPD medium and shake cultured at 30°C for 12-20 hours. The cells were then diluted to an $A_{600}$ of about 0.01 to 0.1 and maintained in log phase in YPD medium at 30°C for about 6-8 hours. 100 ml of YPD medium were inoculated with 0.5 ml of the seed culture at an $A_{600}$ of about 0.1 and shake cultured at 30°C for about 12-20 hours. The culture was then harvested when $A_{600}$ was about 0.2 to 0.3 by centrifugation using a DAMON IEC DPR-6000 centrifuge at 1500 g for 5 minutes.

To prepare spheroplasts, the cells were washed once in 10 ml of sterile water (centrifugation was performed after each wash as described above), once in 10 ml of freshly prepared SED, once in 10 ml of sterile 1M sorbitol, and resuspended in 5 ml of SCE buffer. 5 μl of 4 mg/ml Zymolyase 60,000 (Miles Laboratories) were added and the cells incubated at 30°C for about 30 minutes.

Spheroplast formation was monitored as follows. 100 μl aliquots of cells were added to 900 μl of 5% SDS and 900 μl of 1M sorbitol before or just after the addition of Zymolyase, and at various times during the incubation period. The incubation was stopped at the point where cells would lyse in SDS but not sorbitol. Once formed, spheroplasts were washed once in 10 ml of sterile 1M sorbitol by centrifugation at 1,000 g for 5-10 minutes, washed once in 10 ml of sterile CaS by centrifugation, and resuspended in 0.6 ml of CaS.

For the actual transformation, DNA samples in water or TE buffer were added (typically 1-10 μg in up to 20 μl total volume) to 1.5 ml Eppendorf microfuge tubes. (For small amounts of DNA, maximum transformation occurs using about 1μl of 5 mg/ml sonicated E. coli DNA in each sample). 100 μl of spheroplasts were added to each DNA sample and incubated at room temperature for about 20 minutes. 1 ml of PEG solution was added to each sample and incubated at room temperature for about 15 minutes, and the samples were plated as described below.

10 ml of Regeneration Agar were poured per plate at least 30 minutes before transformation samples were ready. 10 ml aliquots of Regeneration Agar were also distributed to tubes in a 45-50°C bath during the period that transformation samples were in SOS. Samples were then added to the tubes, poured onto plates containing the solid bottom agar layer, and incubated at 30°C for 3-5 days.

Spheroplast quality was determined as follows. 10 μl of sample were removed and diluted 100 X by addition to 990 μl of 1M sorbitol. 10 μl of the dilution were removed, and an additional 990 μl aliquot of 1M sorbitol was added. 100 μl of both dilutions were spread on YPD agar plates to determine the concentration of unspheroplasted whole cells remaining in the preparation. To determine the total regeneratable spheroplasts, 100 μl of each dilution were added to 10ml of Regeneration Agar which had been supplemented with 40 μg/ml of all amino acids required by the host. Good values for a transformation experiment were 1-3 X $10^7$ total regenerable spheroplasts/ml and about 1 X $10^3$ whole cells/ml.

12

## Example III

### Yeast DNA Preparation

Yeast cells were grown in 100 ml of YNB medium plus 2% dextrose at 30° C until $A_{600}$ equaled 1-2 and then pelleted using a Damon IEC DPR-6000 centrifuge 15 2,000 g for 5 minutes. The pellet was washed once in dH₂0, once in SED, once in 1M sorbitol and then resuspended in 5 ml of a solution of 0.1M tris•Cl, pH 7.0, 1M sorbitol and 10 mM EDTA. The cells were then mixed with 50-100 μl of a 4 mg/ml solution of Zymolyase 60,000 (Miles Laboratories) and incubated at 30° C for 1 hour. The resulting spheroplasts were then centrifuged at 1,000 g for 5-10 minutes and suspended in 5 ml Lysis Buffer [0.1% SDS, 10mM Tris•Cl (pH 7.4), 5mM EDTA and 50mM NaCl]. Proteinase K (Boehringer Mannheim) and RNase A (Sigma) were each added to 100 μg/ml and the solution incubated at 37° C for 30 minutes. DNA was deproteinized by gently mixing the preparation with an equal volume of chloroform containing isoamyl alcohol (24:1, v/v), and the phases were separated by centrifugation at 12,000 g for 20 minutes. The upper (aqueous) phase was drawn off into a fresh tube and extracted with an equal volume of a 50/48/2 mixture of phenol/chloroform/isoamyl alcohol. The phases were separated as before and the top phase placed in a tube containing 2-3 volumes of cold 100% ethanol. The sample was gently mixed and DNA was collected by spooling onto a plastic rod. The DNA was immediately dissolved in 1 mL of TE buffer and dialyzed overnight at 4° C against 100 volumes TE buffer.

## Example IV

### Construction of pRA110

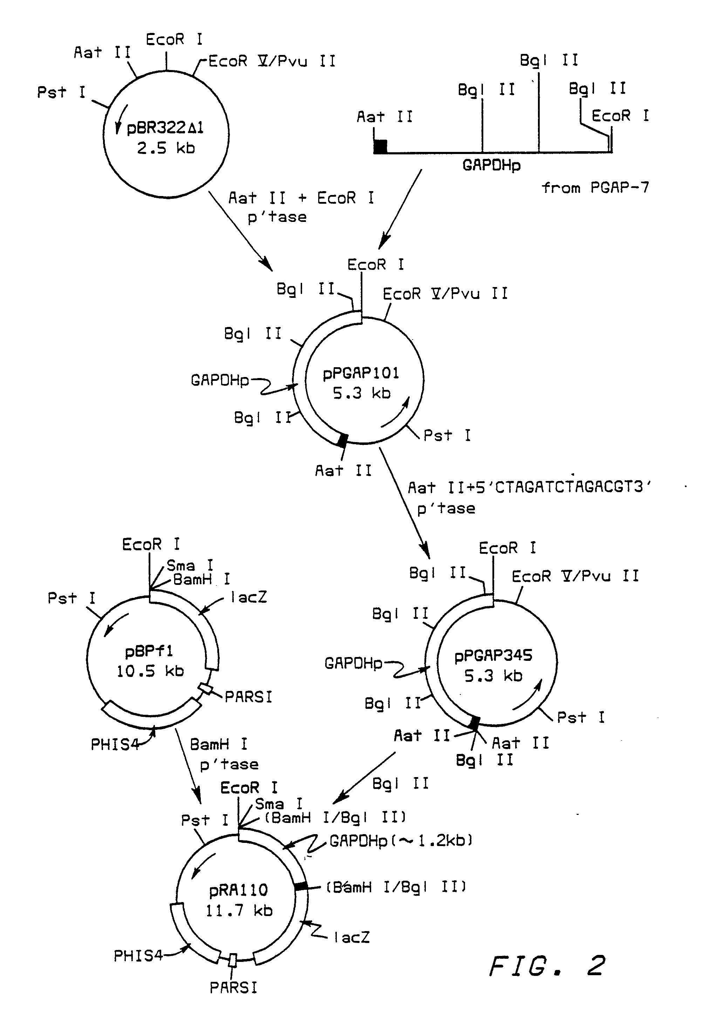

10μg of pBR322 were digested with EcoRV and PvuII following manufacturer's instructions. The plasmid was ligated using 0.5 units of T4 DNA ligase in 66mM Tris•Cl, pH 7.4, 6.6mM MgCl₂, 10mM DTT, and 0.4 mM ATP for 24 hours at 4° C. This was designated pBR322Δ1. 20 μg of pGAP-7 (from Example I) was digested with AatII and EcoRI. A 2.85 kb fragment containing the 5′ non-coding sequence and amino-terminal coding sequence of the Pichia pastoris GAPDH gene was isolated from a 0.8% agarose gel. 10 μg of pBR322Δ1 was digested with AatII and EcoRI and dephosphorylated using alkaline phosphatase in a 100 μl reaction volume according to manufacturer's instructions. 3.3 μg of the AatII - EcoRI fragment and 100 ng of the plasmid were ligated as above. The resulting plasmid was designated pPGAP101. 10 μg of pPGAP101 was digested with AatII and dephosphorylated as above. A new BglII site was created in pPGAP101 as follows. An adapter oligonucleotide was synthesized using an Applied Biosystems DNA Synthesizer, Model 380A, using cyanoethylphosphoramidite chemistry:
5′-CTAGATCTAGACGT-3′
500 ng of the oligonucleotide were annealed by heating to 80° C, allowed to slow cool, and then kinased (see Maniatis et al.; 1982) 30 ng of the annealed adapter and 10 ng of the AatII-digested pPGAP101 were ligated using T4 ligase as described above. The resulting plasmid was designated pPGAP345. 20 μg of pPGAP345 were digested with BglII. A 1.2 kb BglII fragment from pPGAP345 was then isolated from a 0.8% agarose gel. 10 μg of pBPf1 (NRRL #B-15892) were digested with BamHI and dephosphorylated as above. 100 ng of the 1.2 kb BglII fragment and 50 ng of the BamHI-digested and phosphatase treated pBPf1 were ligated as above. The resulting plasmid was designated pRA110, and contained the above-described portions of the Pichia pastoris GAPDH gene fused in frame to the E. coli β-galctosidase gene from pBPf1. Construction of pRA110 is detailed in Figure 2.

## Example V

### Preparation of Strain GS115(pRA110)

Pichia pastoris GS115, a histidine auxotroph, (NRRL Y-15851; his4) was transformed with plasmid pRA110 (from Example IV) as described in Example II. Prior to transformation, pRA110 was digested with BamHI to facilitate integration of the plasmid into the host genome. Genomic DNAs from individual, stable His⁻ transformants were analyzed by Southern filter hybridization to determine the genomic location of the plasmid. 1.5 μg of genomic DNA was prepared according to Example III. This DNA was digested with BglII, subjected to electrophoresis using 0.7% agarose gels, and transferred to nitrocellulose filters [Southern, J. Mol. Bio. 98, 503 (1975)]. A probe complementary to the P. pastoris HIS4 locus (pYM4) and a probe complementary to the P. pastoris GAPDH locus (pRA106) were labeled by nick translation and used to determine the site of integration of the pRA110 plasmid. [The pRA106 plasmid was formed by ligating 100 ng of SalI-digested and alkaline phosphatase-treated pUC19 DNA with 200 ng of the 700 bp SalI fragment isolated from 20 μg of SalI-cut pPGAP-6 DNA after electrophoresis through 0.8% agarose.] [Plasmid pYM4 can be obtained by digesting pYM30 (NRRL #B-15890) with ClaI and religating the ends.] One transformant containing pRA110 integrated at the GAPDH locus was designated GS115(pRA110).

### Example VI

### Product of b-Galactosidase Fusion Protein Using P. pastoris GAPDH Promoter Plus 5′ Coding Sequence

Regulation and expression of the GAPDH promoter has been characterized by determining the β-galactosidase activity in the strain GS115 (pRA110). 250 ml cultures were grown in YNB medium and either 2% glucose or 0.5% methanol at 30°C. The culture was diluted at regular intervals to maintain cells in logarithmic growth. Samples of 25 OD units of cells (~1.5 X 90⁹ cells) were removed at various times during logarithmic growth of the culture and frozen at -20°C.

β-galactosidase activity was determined as follows.

### β-Galactosidase Assay

1) Solution Required:

| Z-buffer, 1 liter | | Final Concentration |
|---|---|---|
| $Na_2HPO_4 \cdot 7H_2O$ | 16.1 g | 0.06 |
| $NaH_2PO_4$ | 5.5 g | 0.04 M |
| KC1 | 0.75 g | 0.01 M |
| $MgSO_4 \cdot 7H_2O$ | 0.246 g | 0.001 M |
| 2-mercaptoethanol | 2.7 mL | 0.05 M |

fill up to 1 L; pH should be 7

0-Nitrophenyl-β-D-galactoside (ONPG):

Dissolve 400 mg ONPG (Sigma N-1127) in 100 mL of distilled water to make a 4 mg/mL ONPG solution

2) Cell-free Protein Extract Preparation

14

Each sample was washed once in dH2O, once in lysis buffer (62.5 mM Tris·Cl, pH 8.7, 5 mM EDTA, 2 mM PMSF), and resuspended in 400 $\mu$l lysis buffer. 0.5 g of 0.45 mm glass beads were added to a 400 $\mu$l aliquot of sample, and the mixture was vortexed four times for 60 seconds each with 1 minute intervals on ice. The cell slurry was removed from the glass beads, and the suspension was centrifuged for 5 minutes in a microfuge. The supernatant was transferred to a polypropylene microfuge tube for assaying. The amount of total protein in each extract was determined by the Lowry assay method. BSA served as the protein standard.

3) Assay Procedure

1 $\mu$l of cell-free protein extract was added to 1 ml of Z buffer. The mixture was then vortexed and incubated for 5 minutes at 30° C. The reaction was initiated by the addition of 0.2 ml of ONPG (4mg/ml). 0.5 ml of a 1M $Na_2CO_3$ solution was added to stop the reaction at an appropriate time, ($A_{420}<1$) usually between 2 and 5 minutes. The absorbance at 420 nm was then read.

4) Calculation of $\beta$-galactosidase Activity Units

1 U = 1 nmole of orthonitrophenol (ONP) formed per minute at 30° C and pH 7. 1 nmole of ONP has an absorbance at 420 nm ($A_{420}$) of 0.0045 with a 1 cm path length. Therefore, an absorbance of 1 at 420 nm represents 222 nmoles ONP/ml, or 378 nmoles ONP/1.7 ml (the total volume of supernatant being analyzed was 1.7 ml). Units expressed in Table 2 were calculated as follows:

$$U = \frac{A_{420}}{t\ (min)}\ X\ 378$$

### Glyceraldehyde-3-Phosphate Dehydrogenase Assay

Glyceraldehyde-3-phosphate dehydrogenase activity was assayed in 1.0 ml reactions containing 0.1 M potassium phosphate (pH 7.4), 1.0 mM $NAD^+$, 10 mM EDTA, 0.1 mM dithiothreitol, and 4.0 mM glyceraldehyde 3-phosphate. NADH formation was monitored spectrophotometrically at 340 nm. Glyceraldehyde-3-phosphate dehydrogenase activity units are expressed as $\mu$mol of NADH formed/mg protein/min. at 25° C [McAllister and Holland, JBC 260, 15017 (1985)].

Table 2

| Enzyme Activities in Cell Extracts From Cells Expressing b-Galactosidase Fusion Constructs Grown in Different Carbon Sources | | | |
|---|---|---|---|
| Strain | Carbon Source | Units of Enzyme Activity* | |
| | | $\beta$-gal x $10^{-3}$ | GAPDH |
| GS115(pRA110) | 2% glucose | 22.5 | 2.4 |
| | 0.5% methanol | 14.5 | 1.6 |

*Enzyme activities are expressed as $\mu$mole product/mg of protein/minute, and represent an average of determinations from three samples of cells in logarithmic growth.

## Example VII

### Construction of pRA104

A new EcoRI site overlapping with the initiating methionine of the Pichia GAPDH gene was created as follows. An oligonucleotide of the following sequence was synthesized using an Applied Biosystems DNA Synthesizer, Model 380A, using cyanoethylphosphoramidite chemistry:

5'-TACCGACAGTGATAGGAATTCTGTTTTGATAGTTGT-3'

The M13 clone, G15 (see Example I), served as the template for mutagenesis. This clone contained the Pichia pastoris GAPDH gene BamHI to SalI fragment located at -494 to +271. Annealing, mutagenic oligonucleotide extension, and alkaline sucrose gradient centrifugation were carried out according to Zoller and Smith, Meth. Enzymol. 100, 468 (1983). Screening of positives was done using solution hybridization and electrophoresis through agarose gels, as described by Hobden et al., Analytical Biochem. 144, 75 (1985). The DNA clone bearing the new EcoRI site was called mp18G15*. The correct location and sequence of the site in the DNA were confirmed by DNA sequencing according to Sanger et al., PNAS 74, 5463 (1977) method. DNA containing this new restriction site was transferred into pBR322-based plasmids by cutting replicative-form DNA from mp18G15* and from plasmid pRA103 (see below) with BamHI and SalI, and combining the large BamHI-SalI piece of pRA103 with the small BamHI-SalI piece from mp18G15*. In pRA103, the approximately 3.6 kb genomic HindIII to SalI fragment extending from -3.25 kb to +.27 kb from the Pichia genomic clone, pPGAP-7, was subcloned into the HindIII to SalI sites of pBR322. The analogous clone bearing the mutagenized BamHI-SalI fragment was designated pRA104.

## Example VIII

### Construction of pSL17

1 μg of pBR322 was digested with SalI, filled in with Klenow polymerase, and ligated. The resulting plasmid was designated pSL14. 14 μg of plasmid pTHBS1 were digested with PstI and EcoRI and an approximately 1.1 kb fragment containing approximately 800 bp of pBR322 DNA and an ~300 bp terminator fragment from the Pichia pastoris AOX1 gene was isolated from a 0.8% preparative agarose gel. This same 300 bp terminator fragment can be isolated as a StuI-HindIII fragment from plasmid pBSAGI5I, which is deposited as NRRL #B-18021, and EcoRI linkers can be added to the StuI site.

9 μg of pSL14 were digested with PstI and EcoRI and dephosphorylated using calf intestinal alkaline phosphatase as described above. 80 ng of the 1.1 kb PstI-EcoRI fragment were ligated to 12.5 ng of the PstI-EcoRI digested pSL14, and the resulting plasmid was designated pSL15.

18 μg of pRA104 (from Example VII) were digested with EcoRI and BamHI and a 515 bp fragment containing the Pichia pastoris GAPDH promoter was isolated from a 5% polyacrylamide gel. 10 μg of pSL15 were digested with BamHI and EcoRI and dephosphorylated using calf intestinal alkaline phosphatase as described above. 50 ng of the EcoRI-BamHI fragment were ligated to 15 ng of the EcoRI-BamHI-digested pSL15. The resulting plasmid was designated pSL16. 10 μg of pSL16 were digested with BamHI and dephosphorylated with alkaline phosphatase, as above.

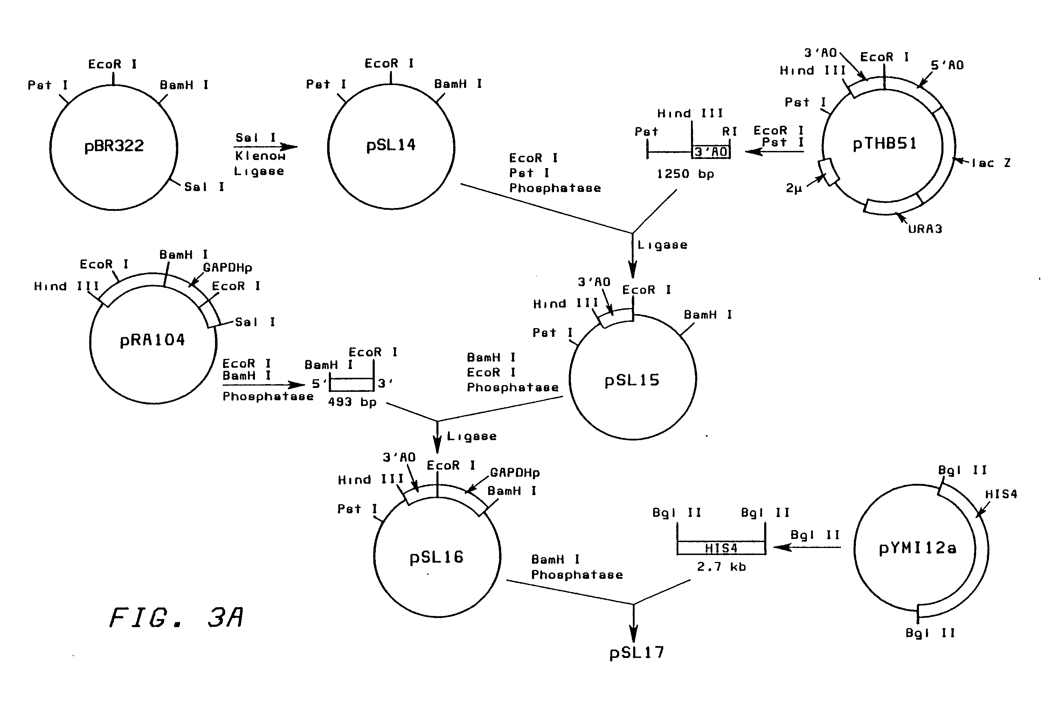

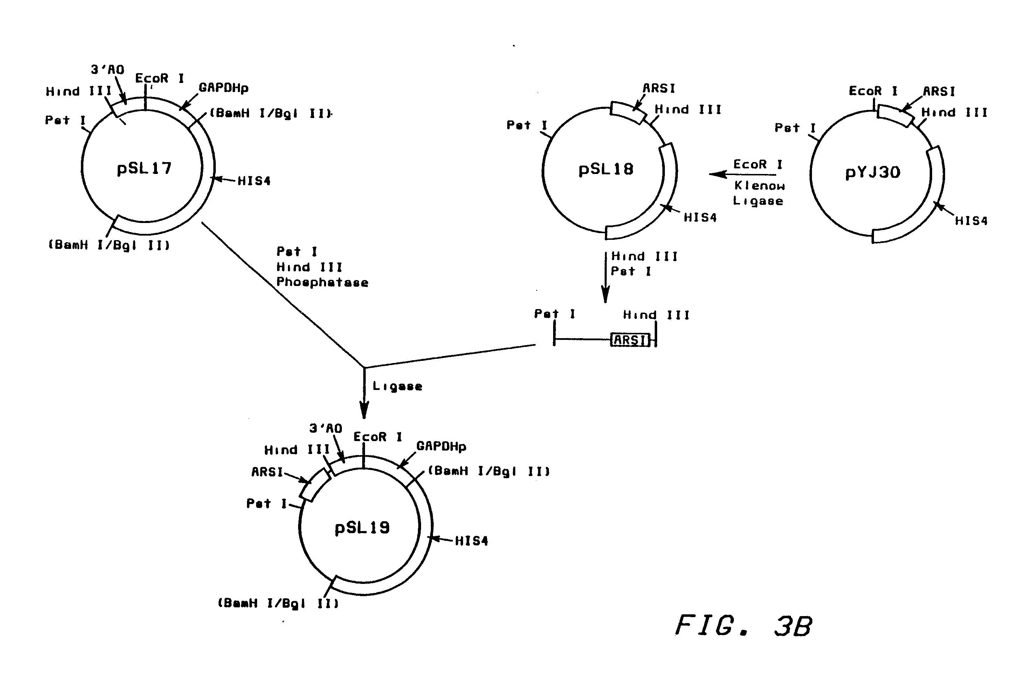

The Pichia pastoris HIS4 gene was added to pSL16 for use as a selectable marker. The approximately 2.7 kb BglII fragment was isolated from the plasmid pYMI12a by cutting 10 μg of pYMI12a with BglII and isolating the 2.7 Kb BglII fragment from a 0.8% agarose gel. This same BglII fragment can be isolated from plasmid pYJ8, which is deposited as NRRL #B-15889. However, the BglII fragment containing the HIS4 gene in plasmid pYMI12a has had a BamHI site which exists within the gene removed by digesting pYJ8 with BamHI and filling in the sticky ends using Klenow polymerase, followed by a blunt-end ligation. The removal of the BamHI site from the BglII fragment containing the HIS4 gene does not affect the HIS4 gene activity of the fragment. 75 ng of the BglII fragment and 25 ng of BamHI-digested, dephosphorylated pSL16 were ligated using T4 ligase as described above. The resulting plasmid was designated pSL17 and is shown in Figure 3B.

## Example IX

## Construction of pSL19

10 μg of pYJ30 (NRRL B-15890) were digested with EcoRI and filled in with Klenow polymerase. 20 ng of this DNA were then ligated with T4 ligase as described previously. This was designated pSL18. 20 μg of pSL18 were digested with PstI and HindIII. A ~900 bp fragment was isolated from a 0.8% agarose gel. 10 μg of pSL17 were digested with PstI and Hind III and dephosphorylated using calf intestinal alkaline phosphatase. 40 ng of the fragment and 10 ng of the PstI-HindIII digested and alkaline phosphatase-treated plasmid were ligated using T4 ligase as described above. The resulting plasmid was designated pSL19. The construction of pSL19 is shown in Figure 3B.

## Examples X

## Construction of pSL20A

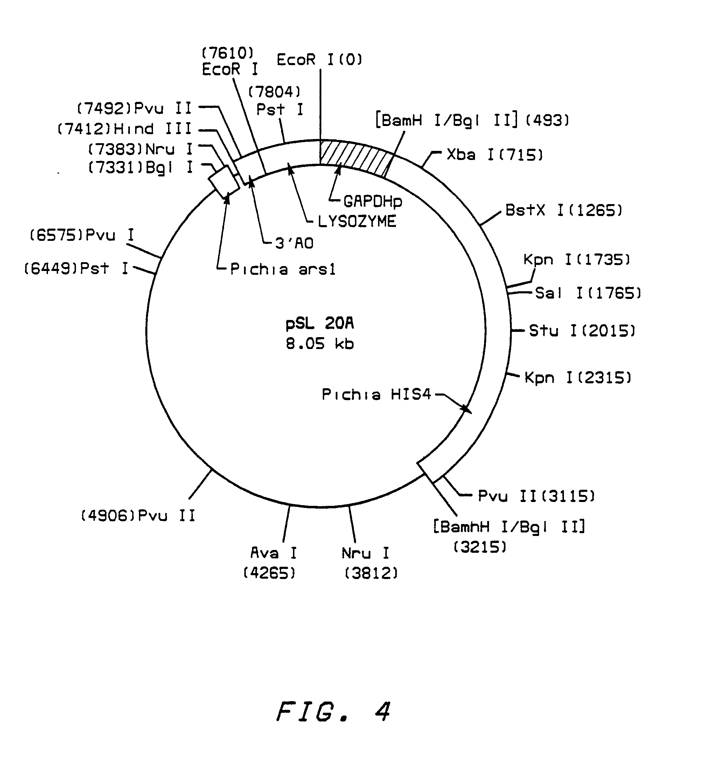

The pSL19 plasmid from Example IX was used to construct an expression plasmid for bovine lysozyme c2, with the sequence of the EcoRI-linkered DNA encoding bovine lysozyme c2 located in Table 3. The EcoRI fragment encoding this protein was purified from the plasmid pSL13, a subclone of this fragment in the pUC19 vector. 10 ng of pSL19 DNA were digested with EcoRI and treated with alkaline phosphatase. 40 ng of the 0.46 Kb EcoRI fragment containing the bovine lysozyme gene were ligated with the pSL19 DNA. The resulting plasmid was designated pSL20A (Figure 4).

DNA from this plasmid was cut with SalI and used to transform P. pastoris GS115 according to Example II. These cells were grown in the fermentor in minimal glucose media, and the effect of dilution rate on the specific productivity of bovine lysozyme was measured. At dilution rates of 0.1 and 0.2 $hr^{-1}$, the specific productivities were approximately 1 μg/g cells/hr, whereas when the dilution rate of the same strain was raised above the maximum specific growth rate to 0.3 $hr^{-1}$, the specific productivity increased ten-fold, to approximately 10 μg/g cells/hr.

## Table 3

1

G A A T T C A T G A A G G C T
EcoRI Adaptor

10                    20                    30
C T C G T T A T T C T G G G G T T T C T C T T C C T T

40                    50                    60
T C T G T C G C T G T C C A A G G C A A G G T C T T T

Mature
Protein
Starts
------

70                    80                    90
G A G A G A T G T G A G C T T G C C A G A A C T C T G

```
          100                 110
A A G A A A C T T G G A C T G G A C G G C T A T A A G


          120                 130                 140
G G A G T C A G C C T G G C A A A C T G G T T G T G T


          150                 160                 170
T T G A C C A A A T G G G A A A G C A G T T A T A A C


             180                 190
A C A A A A G C T A C A A A C T A C A A T C C T A G C


200                 210                 220
A G T G A A A G C A C T G A T T A T G G G A T A T T T


          230                 240                 250
C A G A T C A A C A G C A A A T G G T G G T G T A A T


             260                 270
G A T G G C A A A A C C C C T A A T G C A G T T G A C


280                 290                 300
   G G C T G T C A T G T A T C C T G C A G C G A A T T A


       310                 320                 330
   A T G G A A A A T G A C A T C G C T A A A G C T G T A


          340                 350                 360
   G C G T G T G C A A A G C A T A T T G T C A G T G A G


             370                 380
C A A G G C A T T A C A G C C T G G G T G G C A T G G


    390                 400                 410
A A A A G T C A T T G T C G A G A C C A T G A C G T C


    420                 430                 440
A G C A G T T A C G T T G A G G G T T G C A C C C T G


             450
```

EP 0 374 913 A1

T A A T T C G A A T T C
EcoRI Adaptor

## Claims

1. An isolated DNA fragment comprising the Pichia pastoris GAPDH 5′ regulatory region.

2. The DNA fragment of claim 1 wherein said DNA fragment has the restriction map of Figure 1(B).

3. The DNA fragment of claim 2 wherein said DNA fragment has the nucleotide sequence from about nucleotide -490 to about nucleotide -1 shown in Table 1.

4. The DNA fragment of claim 1 wherein said DNA fragment has the restriction map of Figure 1(C).

5. The DNA fragment of claims 2 or 4 wherein said DNA fragment is operably linked to a heterologous DNA sequence encoding a polypeptide.

6. The DNA fragment of claims 2 or 4 wherein said DNA fragment is operably linked to a heterologous DNA sequence selected from streptokinase, tissue plasminogen activator, hepatitis surface antigen and bovine lysozyme.

7. The DNA fragment of claim 5 wherein said DNA fragment is linked to a DNA sequence capable of replicating in cells selected from prokaryotic and eukaryotic cells.

8. A method for activating the GAPDH 5′ regulatory region to facilitate the high level expression of a heterologous DNA sequence, which encode at least one polypeptide comprising contacting a suitable host cell which has been transformed with the GAPDH 5′ regulatory region operably linked to a heterologous DNA sequence, with a compound selected from glucose, glycerol and methanol or combinations thereof in a manner to facilitate the host cell's ingestion of said compound under conditions suitable for growth of the host cell.

9. An isolated DNA fragment comprising the Pichia pastoris GAPDH 3′ transcription termination sequence.

10. The DNA fragment of claim 9 wherein said DNA fragment has the restriction map of Figure 1(E).

11. The DNA fragment of claim 9 wherein said DNA fragment has the nucleotide sequence of from about nucleotide 1005 to about nucleotide 1380 as shown in Table 1.

12. The DNA fragment of claim 10 wherein said DNA fragment is operably linked to a heterologous DNA sequence which encodes for at least one polypeptide.

13. An isolated DNA fragment comprising the Pichia pastoris GAPDH structural gene.

14. The DNA fragment of claim 13 wherein said DNA fragment has the restriction map of Figure 1(D).

15. An isolated DNA fragment comprising the Pichia pastoris GAPDH gene.

20

FIG. 1

FIG. 2

FIG. 3A

FIG. 3B

EP 0 374 913 A1

*FIG. 4*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 263 311 (PHILLIPS PETROLEUM CO.) <br> * Claim 2 * <br> --- | 1-8 | C 12 N 15/81 <br> C 12 N 15/53 <br> C 12 P 21/02 // <br> (C 12 N 15/81 <br> C 12 R 1:84 ) |
| A | EP-A-0 188 677 (PHILLIPS PETROLEUM CO.) <br> --- | | |
| A | EP-A-0 129 268 (UNILEVER) <br> --- | | |
| A | GENE, vol. 69, no. 1, pages 49-57, Elsevier Science Publishers B.V., Amsterdam, NL; P.J. PUNT et al.: "Isolation and characterization of the glyceraldehyde-3-phosphate dehydrogenase gene of Aspergillus nidulans" <br> ----- | | |

TECHNICAL FIELDS SEARCHED (Int. Cl.5)

C 12 N
C 12 P

The present search report has been drawn up for all claims

| Place of search | Date of completion of the search | Examiner |
|---|---|---|
| THE HAGUE | 06-03-1990 | VAN PUTTEN A.J. |